# EUROPÄISCHE PATENTSCHRIFT

(11) **EP 1 781 330 B1**
(45) Veröffentlichungstag und Bekanntmachung des Hinweises auf die Patenterteilung: **27.03.2013**
(21) Anmeldenummer: 05768354.2
(22) Anmeldetag: 26.07.2005
(51) Int. Cl.: A61K 45/00, A61K 31/198, A61K 36/00, A61K 36/16, A61K 36/896, A23L 1/30, A23L 1/302, A23L 1/304, A23L 1/305

(54) **EIN KAPILLAR- WIRKSYSTEM ENTHALTENDE ZUSAMMENSETZUNGEN MIT ANWENDUNGSBEZOGENER DIFFERENZIERBARKEIT UND DEREN VERWENDUNG**
COMPOSITIONS CONTAINING A CAPILLARY ACTIVE SYSTEM WITH APPLICATION-RELATED DIFFERENTIABILITY, AND THE USE THEREOF
COMPOSITIONS CONTENANT UN SYSTEME ACTIF CAPILLAIRE A DIFFERENTIABILITE D'APPLICATION ET UTILISATION

(30) Priorität: 26.08.2004 DE 102004041270
(43) Veröffentlichungstag der Anmeldung: 09.05.2007
(60) Teilanmeldung: 10009259.2
(73) Patentinhaber: Merz Pharma GmbH & Co. KGaA, 60318 Frankfurt (DE)
(72) Erfinder: BEUTLER, Rolf, D., 64739 Höchst/Hummetroth (DE); SCHMIDT, Karl-Heinz, 72810 Gomaringen (DE); FUNKE, Bettina, 60316 Frankfurt (DE); BECKER, Kirstin, 60316 Frankfurt (DE)
(74) Vertreter: Müller, Claudia
(86) Internationale Anmeldenummer: PCT/EP2005/008087
(87) Internationale Veröffentlichungsnummer: WO 2006/021278

(56) Entgegenhaltungen:
- WO-A-01/09000
- DE-U1- 20 204 843
- DE-U1- 20 309 608
- GB-A- 2 268 871
- NZ-A- 530 554
- US-A1- 2002 119 933
- US-A1- 2002 183 263
- US-A1- 2003 003 162
- US-A1- 2003 008 048
- US-A1- 2003 044 473
- US-A1- 2003 224 071
- US-A1- 2004 151 783
- US-B1- 6 462 051
- US-B1- 6 572 899

## Beschreibung

Ein Kapillar- Wirksystem enthaltende Zusammensetzungen mit anwendungsbezogener Differenzierbarkeit und deren Verwendung

### Gegenstand der Anmeldung

Die vorliegende Erfindung betrifft Zusammensetzungen zur oralen Anwendung gemäß Anspruch 1 mit einem Kapillar-Wirksystem mit anwendungsbezogener Differenzierbarkeit und synergistischem Potential für die Verwendung bei Säugern, vor allem Menschen.

Die Erfindung betrifft insofern eine Zusammensetzung, welche ein Kapillar-Wirksystem K aufweist aus einem Kapillar - Aktivatorsystem KA aus Blutfluss steigernden, Gefäßwand stabilisierenden und/oder Gefäß erweiternden Mitteln; und einem Kapillar- Protektorsystem KP, umfassend Mittel zur Endothelstabilisierung, zum Lipoproteinschutz, sowie zur Leukozyten-/Thrombozytenstabilisierung; und einem Kapillar- Energieversorgungssystem KE, umfassend Redoxsysteme und Cofaktoren bei der Energiebereitstellung sowie Energieträger.

Dieses System K wird mit einem selektiven Wirksystem S kombiniert.

Die Erfindung betrifft ferner die Verwendung derartiger Zusammensetzungen, vor allem zur selektiven Beeinflussung von z. B. strukturellen Veränderungen, funktionellen Störungen der betreffenden Zielorgane (Haare, Haut, Cerebrum, Immunsystem, Muskeln, Gastro-Intestinaltrakt), als Supplemente (Nahrungsergänzungsmittel, Ergänzende bilanzierte Diät, Diätetikum), oder pharmazeutisches Mittel.

### Stand der Technik

Zur Versorgung einzelner Organe werden Arzneimittel eingesetzt. Diese zielen vor allem auf die Organ spezifische Wirkung einzelner Substanzen ab, wobei durch diese selbst über Transport / Verweilzeit entschieden wird.

Daneben sind auch insbesondere Supplemente wie Nahrungsergänzungsmittel (Mikronährstoffpräparate) bekannt. Dabei handelt es sich zum einen um Produkte mit einer Reihe unterschiedlichster Inhaltsstoffe, mit welchen möglichst breit verschiedene Bedürfnisse des Organismus abgedeckt werden sollen (z. B. Multivitaminpräparate). Dabei bleibt unklar, ob tatsächlich ein derartig breites Wirkungsspektrum aktiv vorhanden ist, oder ob beabsichtigte Wirkungen nicht gegenseitig aufgrund der Fülle an unterschiedlichen Wirkgruppen aufgehoben werden.

Aus diesem Grund werden auch Produkte eingesetzt, welche jeweils nur einen Wirkstoff für eine bestimmte Anwendung besitzen (z. B. Monovitaminpräparate). Damit muss für die jeweiligen unterschiedlichen Substanzen auch jeweils ein entsprechendes System bereitet werden, damit der beabsichtigte Zweck auch erreicht wird. Dieses kann auch von einer bestimmten Darreichungsform abhängen. So beschreibt z. B. die DE 43 26 698 C2 die Verwendung einer Vitamin B6 / B12 / Folsäure- Kombination insbesondere als Injektion zur Prophylaxe / Therapie von Arteriosklerose. Selbige Kombination wird nach der DE 43 26 675 C2 zur Vorbeugung von Nervendegenerationserkrankungen und Altersdementia eingesetzt. In der DE 100 22 510 A1 werden Mittel, enthaltend Folsäure, 5-Methyltetrahydrofolsäure und/oder 5-Methyltetrahydrofolsäurepolyglutamat als Nahrungsergänzungsmittel beschrieben.

In der DE 102 06 159 A1 sind Mittel, enthaltend spezielle Kombinationen aus Folsäure, Vitamin B6, B12 und deren Verwendung zur Senkung von Homocysteinspiegeln offenbart.

In der DE 100 35 088 C2 wird ein Vitamin C Präparat offenbart, das in einer besonderen Form, nämlich als Bläschen oder Kügelchen mit einem Durchmesser von 0,2 mm, vorliegt.

Die DE 198 58 372 A1 betrifft ein Vitaminpräparat, umfassend ein Trägermaterial aus einem Zucker in Form eines Pulvers, auf das ein Vitamin aufgebracht ist, sowie ein Bindemittel in kristalliner, agglomerierter Form. Durch diese Form des Bindemittels soll es zu einem schnellen Auflösen beim Kontakt mit einer geringen Flüssigkeitsmenge kommen.

Aus der DE 100 16 313 A1 sind pharmazeutische Zubereitungen zur oralen oder peroralen Applikation bekannt, die in Form eines Gels mit einer Retardierung der Wirkstofffreisetzung im Magen-Darm Trakt freigesetzt werden.

Die EP B 0 531 155 beschreibt die Verwendung von Johannisbeerensaft ohne Obstkernöl oder ungesättigte Fettsäuren zur Förderung der Monoamin- Oxidase Inhibition.

DE GM 201 16 346 betrifft ein Mikronährstoffkombinationsprodukt mit bestimmten Mengen an Vitaminen wie 350 bis 700 mg Vitamin C, etc. und Carotinoiden (2-10 mg), welche in getrennten Verabreichungsformen wie Vitamin C- Filmtablette + getrennte Carotinoid- Kapseln vorliegen. Damit soll Mangelerscheinungen begegnet und insbesondere auch keine Verstärkung von Nebenwirkungen bei gleichzeitiger Arzneimittelgabe hervorgerufen werden.

In der EP B 0 796 080 wird ein spezielles Kombinationspräparat zur Förderung des Haar- und Haut-/Nagelwachstums beschrieben, welches ebenfalls Vitamin C in relativ hohen Mengen (15,61%) und weitere Vitamine /Inhaltsstoffe in bestimmten Mengenanteilen aufweist.

EP B 1 001 685 betrifft Nahrungszusammensetzungen, enthaltend Kohlenhydrate, Fette und Eiweiß sowie insbesondere eine bestimmte Menge an Methionin/ Cystein. In der GB A 2 292 522 wird ein Multivitaminpräparat beschrieben, welches Proteine sowie eine Mischung aus Vitaminen sowie Emulgatoren aufweist und zur Herbeiführung eines normal funktionierenden Immunsystems geeignet ist.

In der Roten Liste@ 2004 wird unter Präparat Nr. 84166 ein Dragee mit Vitaminen wie Vitamin A, C, Calciumpanthothenat etc. sowie u.a. Methionin beschrieben.

Andere Multipräparate mit unterschiedlichen Kombinationen für verschiedene Ziele sind z.B. in der US 2002/0183263 A1 (Nahrungsergänzungsmittel auf Ribose-Basis); US 2004/0151783 A1 (Hyperglykämisches Mittel auf Basis eines Ethanol-Extraktes von Gymenma Sylvestre mit einer mittleren Molmasse von wenigstens 3000 Da); GB 2268871 A (multifunktionelles Nahrungsergänzungsmittel auf Proteinbasis mit Aminosäure-/Vitamin- und Mineralstoffgemischen); US 2003/0008048 A1 (Diätetisches Nahrungsergänzungsmittel auf Basis verschiedener Fruchtextrakte, Vitamin C, Grüntee, Schokolade) beschrieben.

Wie hieraus ersichtlich, werden entweder oder nur einzelne Wirkstoffe eingesetzt oder eine Mehrzahl hiervon kombiniert, wobei dann insbesondere auch galenische Maßnahmen hinsichtlich der Wirkstoffe selbst oder bzgl. der damit verbundenen Begleitmaterialien ergriffen werden, um die Stabilität, das Auflösungsvermögen oder die Freisetzungsgeschwindigkeit zu regulieren. Derartige Maßnahmen beziehen sich also auf die physikalischen Eigenschaften der Darreichungsform selbst und sind weitest gehend somit auch von den eingesetzten Wirkstoffen abhängig. Damit müssen je nach Wirkstoff immer neue oder abgewandelte Applikationsformen entwickelt werden, wobei diese entweder nur auf einen Wirkstoff bzw. eine spezielle Wirkstoffgruppe oder eine große Menge unterschiedlicher Wirkstoffe ohne selektive Differenzierbarkeit abgestellt werden, d.h. ohne dass ein generelles Anwendungskonzept vorliegt.

### Aufgabe vorliegender Erfindung

Aufgabe vorliegender Erfindung ist es daher, den oben beschriebenen Mängeln abzuhelfen. Es soll ein Konzept entwickelt werden, wodurch mit Hilfe eines einzigen Systems - also Moduls - ein multidifferenzierbarer Wirkstofftransport auf insbesondere systemisch bzw. enteralem Weg zu einer rationalen, schnellen und vor allem selektiven Übertragung von Wirkstoffen auf dafür bestimmte Ziele bzw. Zielorgane erfolgt, ohne dass für die jeweiligen Wirkstoffe jeweils eine eigene galenische Formulierung entwickelt werden müsste.

### Lösung der Aufgabe

Diese Aufgabe wird erfindungsgemäß dadurch gelöst, dass man neben einem spezifischen Wirksystem S ein multidifferenzierbares Kapillar- Wirksystem K bereitstellt aus einem Kapillar- Aktivatorsystem KA, einem Kapillar- Protektorsystem KP und einem Kapillar- Energieversorgungssystem KE, jeweils umfassend eine oder mehrere der nachfolgend hierzu genannten Mittel.

Erfindungsgemäß wird somit ein generell einsetzbares Modul erhalten, das auf vielfältige Weise je nach gewünschtem Zielorgan eingesetzt werden kann.

Mit Hilfe eines derartigen Systems ist es möglich, eine anwendungsbezogene erhöhte Effizienz insbesondere aufgrund eines schnelleren und rationaleren Transports durch Einsatz des beschriebenen Kapillar Wirksystem K zu erreichen. Dadurch kann es auch ermöglicht werden, dass die aktiven Mittel rationaler am Zielort (Zielorgan) wirken, so dass auch die Anwendungsdosis bei systemisch bzw. enteraler Verabreichung, erniedrigt werden kann.

Insgesamt wird so Wirksamkeit in Abhängigkeit vom Ziel(Organ) multifunktionell - selektiv gesteuert. Damit ist vor allem eine Beeinflussung oder Behandlung von Haut/ -Anhangsgebilde (Haar, Nägel), also des keratinösen Systems, oder auch des immunologischen, ferner des cerebralen sowie des Muskelsystems-, oder des Gatrointestinaltraktes wirksam und unabhängig voneinander möglich. Erfindungsgemäß wird somit erstmals ein System zur Verfügung gestellt, das nicht auf die besondere Be- und Verarbeitung der speziellen aktiven Wirkstoffe ausgerichtet ist, sondern vielmehr unabhängig davon eine verbesserte Versorgung bzw. Beeinflussung bedürftiger Zielorgane unabhängig voneinander durch Aktivierung des Kapillarsystems ermöglicht.

Die erfindungsgemäße Zusammensetzung ist somit von der Darreichungsform unabhängig und ist zur oralen Anwendung konzipiert.

### Beschreibung der Erfindung

Die Erfindung betrifft insbesondere eine Zusammensetzung zur oralen Anwendung mit einer anwendungsbezogenen Wirkstofidifierenzierbarkeit gemäß Anspruch 1. Diese Zusammensetzung besteht aus :
1.) einem Kapillar- Wirksystem K aus einem
   a) Kapillar - Aktivatorsystem KA, umfassend ein oder mehrere Mittel, die ausgewählt sind aus einer oder mehreren Gruppen aus
      KA 1) Blutfluss steigernden Mitteln
      KA 2) Gefäßwand stabilisierenden Mitteln;
      KA 3) Gefäß erweiternden Mitteln
      nämlich: aus Ginkgo-Extrakt, Mäusedorn-Extrakt, L-Arginin-Hydrochlorid;
      und einem
   b) Kapillar-Protektorsystem KP, umfassend ein oder mehrere Mittel, die ausgewählt sind aus einer oder mehreren Gruppen aus
      KP 1) Mitteln zur Endothelstabilisierung;
      KP 2) Mitteln zum Lipoproteinschutz;
      KP 3) Mitteln zur Leukozyten- / Thrombozytenstabilisierung,
      nämlich aus Folsäure, Pyridoxinhydrochlorid, Cyanocobalamin, Tocopherylacetat, omega-3-und 6-Fettsäure-haöltigen Ölen
      und einem
   c) Kapillar- Energieversorgungssystem KE, umfassend ein oder mehrere Mittel, die ausgewählt sind aus einer oder mehreren Gruppen aus
      KE 1) Redoxsystemen bei der Energiebereitstellung;
      KE 2) Cofaktoren bei der Energiebereitstellung;
      KE 3) Energieträgern;
      nämlich Nicotinsäure und dessen Derivate, insbesondere Nicotinsäureamid, Magnesiumoxid, Co-Enzym Q10, Coffein, Glukose;
      und
II.) ein spezifisches Wirksystem S, ausgewählt aus Mitteln der Gruppe, umfassend Haut- und -Anhangsgebilde beeinflussende Mittel S1, cerebral wirksame Mittel S2, immunologisch wirksame Mittel S3, Muskelwirksame Substanzen S5, den Gastrointestinaltrakt beeinflussende Substanzen S6, wie nachfolgend genannt,
   sowie
III.) 0,1 bis 90% Zusatzstoffe, insbesondere galenisch /pharmazeutische Zusatzstoffe.

Mit Hilfe einer solchen Zusammensetzung ist es möglich, insbesondere bei Kombination mit bestimmten Substanzen oder Substanzgruppen (Wirkstoffe, Nährstoffe, pharmazeutische Wirkstoffen) jeweils eine anwendungsbezogene Wirkung zu erzielen, die gegenüber den beschriebenen Multivitamin- Nährstoffpräparaten sehr viel selektiver / effektiver und gegenüber monofunktionellen Produkten sehr viel effektiver ist, da durch das Kapillar -Wirksystem K eine erhöhte Kapillaraktivität und eine gesteigerte Bedarfslage entwickelt wird. Dadurch können die Wirksubstanzen in höherem Ausmaß spezifisch transportiert und eine verbesserte Aktivität bewirkt werden. Dies war insbesondere deshalb überraschend, weil zwar einzelne Komponenten des Kapillar- Wirksystems K an sich bekannt waren, jedoch nicht erwartet werden konnte, dass mit der speziellen Kombination gezielt unterschiedliche Organe selektiv bei erhöhter Bedarfslage angesteuert werden können.

Dabei können unterschiedlichste Darreichungsformen eingesetzt werden. Hierzu gehören insbesondere orale Mittel wie Dragees, Tabletten, Kapseln, etc. Die erfindungsgemäße Zusammensetzung ist somit für die orale Anwendung geeignet. Überraschender Weise ist es hierbei nicht erforderlich, eine besondere Darreichungsform in Abhängigkeit von dem oder den spezifischen Wirkstoffen zu entwickeln, da aufgrund des Wirksystems K allgemein eine Verbesserung der Effizienz unterschiedlicher spezieller Wirkstoffe des Systems S möglich ist.

Die speziellen Wirkstoffe werden dabei ausgewählt aus
S1) Haut - und -Anhangsgebilde beeinflussenden Substanzen;
S 2) Cerebral wirksamen Substanzen;
S 3) Immunologisch wirksamen Substanzen;
S 5) Muskelwirksamen Substanzen;
S 6) Gastrointestinal beeinflussenden Substanzen.;

Die Zusammensetzungen eignen sich daher insbesondere zur Beeinflussung von strukturellen Veränderungen, Störungen der betreffenden Zielorgane und sind zur oralen Applikation geeignet. In der erfindungsgemäßen Zusammensetzung können die einzelnen Systeme der Gruppe K in einer Menge von 0,001 bis 80 %, bevorzugt 0,001 bis 50%, insbesondere 0,01 bis 25%, bezogen jeweils auf KA, KP, oder KE, wobei die Summe an KA + KP + KE bis 98%, vorzugsweise bis 80% betragen kann; 0,1 bis 90%, vorzugsweise bis 65%, insbesondere bis 45% Zusatzstoffe und 0,001 bis 50%, insbesondere 0,001 bis 25% spezifisches System S vorliegen.

Die Mengenangaben beziehen sich auf das Gesamtgewicht der Zusammensetzung. Mit der erfindungsgemäßen Zusammensetzung werden somit 2 Systeme in besonders wirksamer Weise zusammen mit Zusatzstoffen miteinander verknüpft, nämlich das allgemeine Kapillar- Wirksystem K, mit Kapillaraktivator KA, Kapillarprotektor KP und Kapillar- Energieversorgungssystem KE , und das spezifische Wirksystem S, mit den entsprechenden Wirksubstanzen für Haut/Haare/ Nägel, Muskel, Verdauung, Immunsystem, Gehirn.

Das Zusammenwirken beider Systeme K +S ist in nachfolgendem Schema dargestellt:

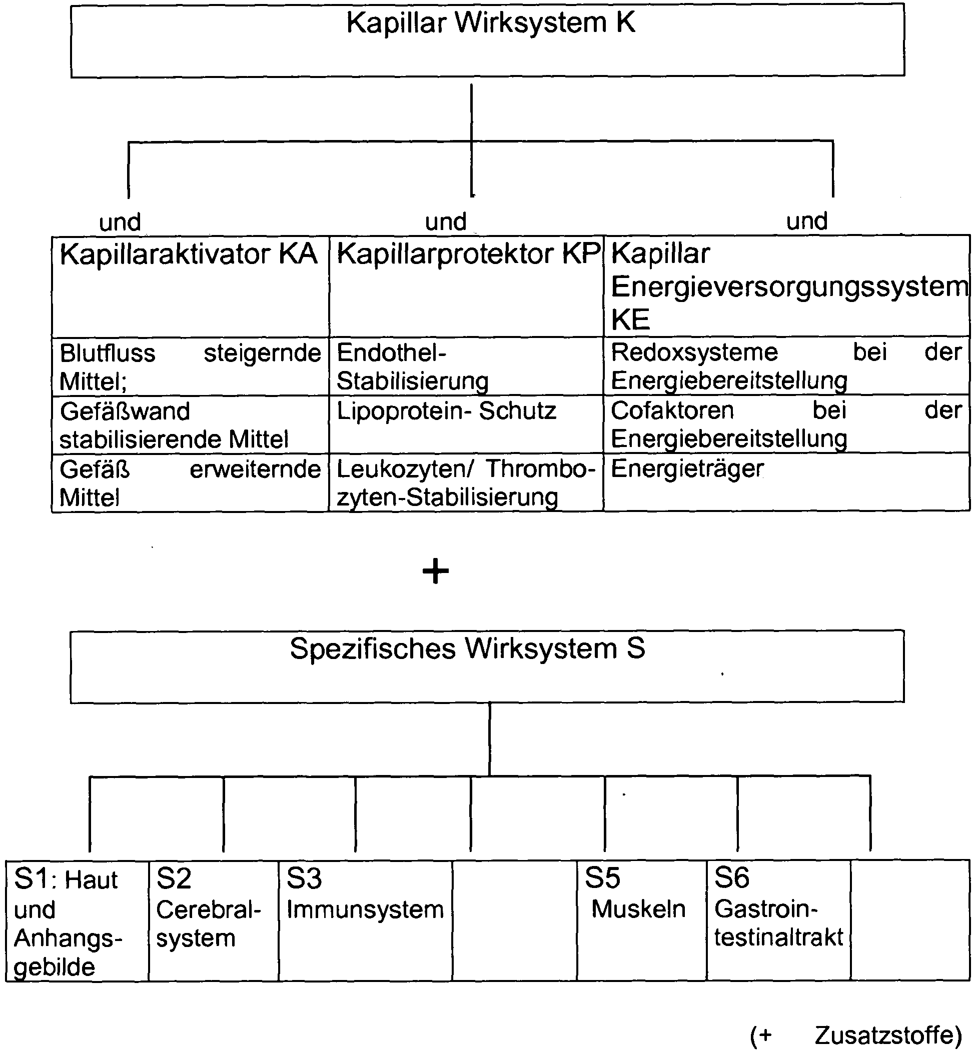

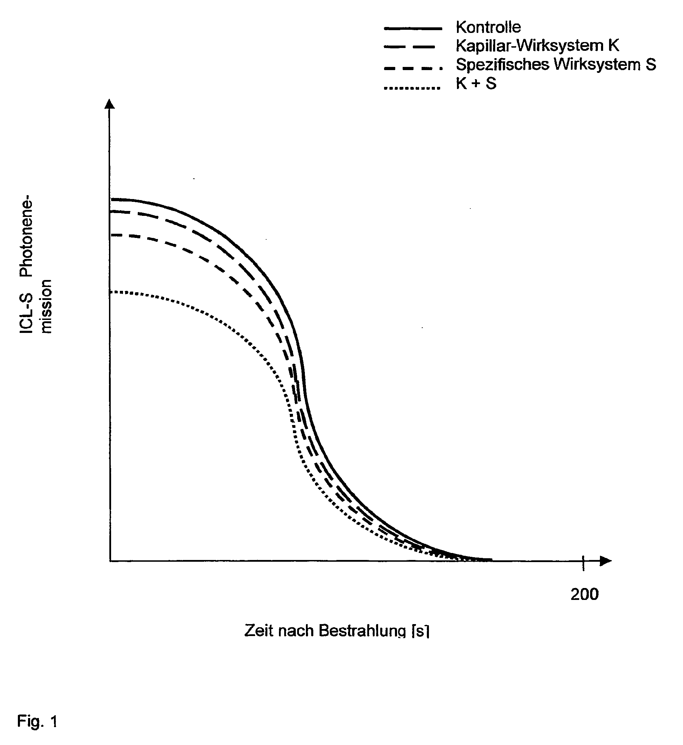

### Beschreibung der Fig. 1

In Fig. 1 ist die besondere Wirksamkeit einer erfindungsgemäßen Zusammensetzung gegenüber Vergleichszusammensetzungen anhand einer ICL-S-Messung aufgezeigt.

### Nähere Erläuterung und bevorzugte Ausführungsformen der Erfindung

### I) Kapillar- Wirksystem K

Die erfindungsgemäße Zusammensetzung umfasst ein Kapillar- Wirksystem K, das ein System aus Ia) +, Ib)-+, Ic) wie beschrieben aufweist.

Das Kapillar -Wirksystem K umfasst ein System KA zusammen mit einem System KP und KE vor.

Dabei hat sich gezeigt, dass das Kapillar -Aktivatorsystem KA aufgrund blutzirkulativer Eigenschaften eine Aktivierung dieses Systems bedingt. Das System KA ist somit agonistisch wirksam und kann selbst überadditive, bis gegebenenfalls synergistische Effekte auf bei Kombination von Mitteln der Gruppe KA 1) + KA 2); KA 1) + KA 3); KA 2) + KA 3) oder KA 1)+2)+3) auslösen.

Das System KP führt insbesondere zu einer Endothelstabilisierung, einem Lipoproteinschutz sowie einer Leukozyten-/Thrombozytenstabilisierung, wodurch zusätzlich Schadwirkungen z. B. durch Oxidantien zurückgedrängt und damit die Regeneration des Endothels ermöglicht wird. System KP ist somit insgesamt als antagonistisch zu bezeichnen.

Das System KE kann schließlich zu einer zusätzlichen Verstärkung durch agonistisches Wirken führen.

Insbesondere kann durch Kombination aus agonistischem System KA (KA1/2/3) und dem antagonistischen System KP oder mit dem antagonistischen System KP und System KE oder durch Kombination von KA/KP/KE, mit einem gewünschten Wirksystem S auch ein überadditiver bis gegebenenfalls synergistischer Effekt erzielt werden. Dieser lässt sich anhand geeigneter Methoden (ICL-S- Messung, Bildgebende Verfahren (Kapillarangiographie, Thermographie), Oximetrie) bzw. der Histaminreagibilität nachweisen. Die besondere Wirksamkeit einer erfindungsgemäßen Zusammensetzung gegenüber Vergleichszusammensetzungen ist anhand eines nachfolgenden Anwendungsbeispiels und in Fig. 1 mittels ICL- S-Messung aufgezeigt.

Die für die einzelnen Systeme KA, KP, KE einzusetzenden Mittel mit den genannten Eigenschaften wie angegeben sind dem Fachmann an sich bekannt. Beispielhaft können die folgenden, insbesondere auch bevorzugten Substanzen genannt werden:
Kapillar - Aktivatorsystem KA:
   Mäusedom-Extrakt, Ginkgo-Extrakt, insbesondere NO- Releaser wie - Arginin und dessen Salze wie L-Arginin-Hydrochlorid . Letzteres bzw. deren Lieferanten, allein oder in Kombination, können insbesondere in einer Menge entsprechend 250 mg L-Arginin vorliegen.
Kapillar -Protektorsystem KP:
   Tocopherol/-acetat (Vitamin E), Folsäure (B₉), Pyridoxin HCL (B₆), Cyanocobalamin (B₁₂), Borretschöl, Leinöl, Traubenkernöl, schwarzes Johannisbeersamenöl, Nachtkerzenöl, Lachsöl, Schwarzkümmelöl (Lieferanten für Ω-3- und Ω-6-Fettsäuren).
Kapillar- Energieversorgungssystem KE:
   Nicotinsäure (Niacin) und dessen geeignete Derivate wie insbesondere Nicotinsäureamid (B₃), Magnesiumoxid / -citrat / -carbonat / -chlorid / gluconat / - phosphat / -lactat / -sulfat,, Q₁₀, Coffein, Glucose.
   Ausführungsformen sind dadurch gekennzeichnet, dass sie ein Kapillar -Aktivatorsystem KA, ein Kapillar- Protektorsystem KP und ein Kapillar-Energieversorgungssystem KE aufweisen.

Ganz besonders bevorzugte Mittel für das System KA sind Arginin, und dessen Salze (z. B. insbesondere wie beschrieben), Ginkgo- Extrakt oder Kombinationen hiervon; für das System KP Folsäure, Cyanocobalamin, Pyridoxin HCL (B₆), Tocopherylacetat oder Kombinationen hiervon; und für das System KE Nicotinsäure (Niacin) und dessen geeignete Derivate wie insbesondere Nicotinsäureamid, Magnesiumoxid; Co Enzym Q10, oder Kombinationen hiervon.

Bevorzugt beträgt die Menge an System KA, KP, KE jeweils 0,001 bis 80 %, bezogen auf das Gesamtgewicht der Zusammensetzung. Insbesondere bevorzugt sind Mengen von 0,01 bis 50%, vor allem 0,01 bis 35%.

Alle Mengen- und Prozentangaben beziehen sich auf das Gewicht der Zusammensetzung, sofern nicht anders angegeben.

Ganz besonders bevorzugt sind weiterhin Zusammensetzungen der beschriebenen Art, worin das Kapillar- Aktivatorsystem KA ein oder mehrere Mittel, ausgewählt aus Ginkgo - Extrakt, Mäusedorn Extrakt, L- Arginin-Hydrochlorid (z. B. in Mengen wie beschrieben); das Kapillar- Protektorsystem KP ein oder mehrere Mittel, ausgewählt aus Folsäure, Pyridoxinhydrochlorid, Cyanocobalamin, Tocopherylacetat, omega-3- und 6-Fettsäurehaltigen Ölen, und das Kapillar-Energieversorgungssystem KE ein oder mehrere Mittel, ausgewählt aus Nicotinsäure und dessen Derivaten, insbesondere Nicotinsäureamid, Magnesiumoxid, Coffein, Glukose, Q10 aufweist bzw. daraus zusammen mit einem spezifischen System S bestehende Zusammensetzungen.

Insbesondere bevorzugt sind Zusammensetzungen, worin das Kapillar Wirksystem K Ginkgo - Extrakt, Folsäure, Magnesiumoxid aufweist.

Nachfolgend sind einige Beispiele für Mittel der Wirksysteme KA, KP und KE in den dafür jeweils spezifischen Mengen angegeben wie z. B. :
- KA:: Gingko-Extrakt, z. B. entsprechend 5 g Droge Mäusedorn- Extrakt: entsprechend 7 - 11 mg Ruscogenine L-Arginin (z. B. xHcl) entsprechend 250 mg L-Arginin
- KP:: Tocopherylacetat: entsprechend 42 mg Vitamin E
Omega-3-Fettsäurehaltiges Öl: entsprechend mind. 90 mg Omega-3-Fettsäuren;
Omega-6-Fettsäurehaltiges Öl: entsprechend mind. 360 mg Omega-6-Fettsäuren;
Folsäure: 0,2 bis 5 mg, bevorzugt 0,2 bis 0,8 mg, vor allem 0,4 mg;
Vitamin B6: 2,0 bis 25 mg; bevorzugt: 2,0 bis 10 mg, insbesondere bis 6 mg, und ganz besonders 2 mg
Vitamin B12: 0,003 bis 0,6mg, insbesondere bis 0,1 mg, vor allem 0,006 mg;

Bevorzugte Mengenbereiche sind hier:

| | | |
|---|---|---|
| Folsäure | 0,4 - 1,2 mg; | Folsäure : Vitamin B₆ = 1 : 1 - 12 |
| Vitamin B₆ | 1,2 - 4,5 mg; | Vitamin B₁₂ = 1 : 0,0025 - 0,0225 |
| Vitamin B₁₂ | 0,003 - 0,009 mg; | Vitamin B₁₂ : Vitamin B₆ = 1 : 40 - 500 |

Bei Mischungen dieser Substanzen kann das Verhältnis Folsäure : Vitamin B₆ = 1 : 5 Folsäure : Vitamin B₁₂ = 1 : 0,015 Vitamin B₁₂ : Vitamin B₆ = 1 : 333 betragen.
- KE:: Nicotinsäureamid: 30 mg Magnesiumoxid/Carbonat: entsprechend 150 mg Magnesium, Coffein, Glukose (500mg);

Ganz besonders bevorzugt wird aus der Gruppe KA L- Arginin- Hydrochlorid, z. B. wie insbesondere beschrieben.

Aus der Gruppe KP ist besonders die Kombination Folsäure, Vitamin B6, Vitamin B12 im Mengenverhältnis 1: 5,0:015 geeignet.
Aus der Gruppe KE werden insbesondere Nicotinsäure/dessen Derivate, insbesondere Nicotinsäureamid und Magnesiumsalze oder Kombinationen hiervon gewählt.

In einer ganz besonders geeigneten Ausführungsform wird ein System KA mit zusammen mit einem System KP mit Folsäure, Vitamin B6 oder Vitamin B12 kombiniert.
Eine weitere geeignete Ausführungsform sieht die oben beschriebene Kombination KA, KP zusammen mit Nicotinsäure, dessen Derivate, insbesondere Nicotinsäureamid, Magnesiumsalz, oder Kombinationen hiervon vor.

Die einzelnen Substanzen aus den genannten Gruppen sind für sich bekannt bzw. können nach dem Fachmann bekannten Verfahren hergestellt werden.

### II) Spezifisches Wirksystem S

Wie erläutert, weist die erfindungsgemäße Zusammensetzung neben dem Kapillar-Wirksystem K weiterhin ein spezifisches Wirksystem S, ausgewählt aus Mitteln der Gruppe, umfassend Haut- und -Anhangsgebilde (Haare, Nägel) beeinflussende Mittel S1, cerebral wirksame Mittel S2, immunologisch wirksame Mittel S3, , Muskelwirksame Mittel S5 , oder den Gastrointestinaltrakt beeinflussende Mittel S6 auf.

Insbesondere bevorzugt sind hierzu Zusammensetzungen, mit einem Kapillar-Aktivatorsystem KA, einem Kapillar-Protektorsystem KP, einem spezifischem Wirksystem S1 oder spezifisches Wirksystem S2 oder S3;, welche zusätzlich dazu noch ein Kapillar Energieträgersystem KE aufweisen.

Dem auf dem Gebiet der Herstellung derartiger Zusammensetzungen zur systemischen bzw. enteralen, insbesondere Lebensmittelprodukten tätigen Fachmann sind die für die oben genannten einzelnen Gruppen jeweils geeigneten Wirkstoffe und die für ihren Einsatz geeignete Mengen bekannt und können demnach je nach Wirkziel ausgesucht und in die erfindungsgemäße Zusammensetzung inkorporiert werden.

Als bevorzugte Mittel des Wirksystems S können die folgenden Substanzen beispielhaft genannt werden:
Haut- und Anhangsgebilde System S1:
   Retinol, Retinolacetat / -palmitat (Vitamin A), Thiaminnitrat (B₁), Biotin, Ca-pantothenat (B₅) (B- Vitamine), Kieselerde, Kieselsäure, Zinkoxid / -sulfat /-gluconat, pflanzliche Extrakte wie Algen, Papaya, Schachtelhalm, Kamille, Aloe vera, Aminosäuren wie Cystein, Methionin, Gammalinolensäure (GLA), Linolsäure; Vitamin C (Ascorbinsäure, Calcium-ascorbat / Natrium-ascorbat, Kalium-ascorbat).
Cerebral - System S2:
   pflanzliche Extrakte wie Ginseng, Paranuß, Wassernabel, Frauenwurz Aminosäuren wie Glutaminsäure, Tyrosin, Tryptophan, Uridin ; Docosahexaensäure (DHA), Phosphatidylserin , Phosphatidylinositol ,Cholin, Lecithin.
Immunologisches System S3:
   Vitamine wie Carotinoide wie Lycopin, Natriumselenat, pflanzliche Extrakte wie Grüntee, Echinacea, Schisandra, Astragalus, Reishi, Shiitake, Maistake, Wiesenklee, Soja, Katzenkralle, Traubenkern, Brokkoli, Tomate, Zwiebel, Blumenkohl, Citrus-Bioflavonoide, Buchweizen, Grapefruit, Aminosäuren wie Threonin, Histidin, Glutathion, Superoxiddismutase.
Muskel - System S5:
   Inositol (B₈), Aminosäuren wie Leucin, Isoleucin, Valin.
Gastrointestinal - System S6:
   α-Linolensäure, Kaliumsalze wie Kalium-carbonat / hydrogencarbonat / -citrat / - chlorid / -gluconat / -lactat / -phosphat,Chromsalze wie Chromchlorid / -picolinat / - sulfat.

Es ist auch von besonderem Vorteil, wenn die erfindungsgemäße Zusammensetzung 1 bis 5, bevorzugt 1 bis 3 Verbindungen des Systems KA; 1 bis 5, bevorzugt 1 bis 3, Verbindungen des Systems KP; 1 bis 5, bevorzugt 1 bis 3, Verbindungen des Systems KE; und / oder 1 bis 5, bevorzugt 1 bis 3 Verbindungen des Systems S aufweist.

Besondere bevorzugte Ausgestaltungen erfindungsgemäßer derartiger Systeme sind Zusammensetzungen, worin als Mittel des Systems S1 eine oder mehrere Substanzen, ausgewählt aus Cystein, Methionin, Biotin, Zinkoxid, Thiaminnitrat (B1), Calciumpantothenat (B5), Kieselerde, als Mittel des Systems S2 Ginseng, Paranuß, Frauenwurz, Aminosäuren wie erläutert oder Mischungen hiervon bzw. als Mittel des Systems S3 pflanzliche Extrakte wie Grüntee, Echinacea, Schisandra, Astragalus, Reishi, Schiitake, Maistake, Wiesenklee, Soja, Katzenkralle, Traubenkern, Brokkoli, Tomate, Zwiebel, Blumenkohl, Citrus - Bioflavonoide, Buchweizen, Grapefruit oder als enthalten sind.
bzw. auch derartige Zusammensetzungen, worin das Aktivatorsystem KA L-Arginin-Hydrochlorid, das Protektorsystem KP Mittel aus der Gruppe KP: Folsäure; Pyridoxin-HCL (B6); Cyanocobalamin (B12); Tocopherol (E); Traubenkernöl, und oder Johannisbeersamenöl und das oder die Mittel der Gruppe KE ausgewählt sind aus Q10, Nicotinsäureamid (Niacin, B3) oder Mischungen hiervon.

Weiterhin bevorzugt sind Zusammensetzungen mit einem System KA+KE+ KP + S5 oder S6.

Insbesondere bevorzugt sind Zusammensetzungen mit Mitteln der Gruppe S1 oder S2 oder S3, welche 0,1 bis 10% Zusatzstoffe aufweisen.

Die einzelnen Wirkstoffe der Gruppe S, deren Herstellung und deren geeignete Mengen sind an sich bekannt.

Es sind auch pharmakologisch wirksame Wirkstoffe möglich, die den einzelnen Gruppen S1, S2, S3, S5 and S6 zugeordnet werden können. Beispielhaft können hierzu genannt werden (wobei auch andere bekannte möglich sind):
Haare: Finasterid, Minoxidil
Haut: Isotretinoin, Prednisolon
Cerebrum: Amantadin, Dopamin
Muskeln: Dantrolen-Natrium, Tetrazepam
Gastro-Intestinaltrakt: Famotidin, Metoclopramid

Die genannten Mittel sind nur beispielhaft und nicht abschließend erwähnt. Der Fachmann kann ggf. die gewünschten Stoffe inkorporieren.

### III. Zusatzstoffe

Die erfindungsgemäße Zusammensetzung kann Zusatzstoffe in einer Menge von 0,1 bis 90 Gew.%, oder bevorzugt 1 bis 90 Gew.%, insbesondere 0,5 bis Gew. 80%, vor allem 2 bis 80 Gew.%, oder 1 bis 50 Gew.%, insbesondere 2 bis 50 Gew.%, bezogen auf das Gesamtgewicht der Zusammensetzung aufweisen, welche aus der Herstellung der genannten Darreichungsformen resultieren. Dazu gehören insbesondere für die enterale, vor allem orale Anwendung: Träger in flüssiger oder fester Form sowie pharmazeutisch zulässige Zusätze oder auch Zusatzstoffe, die für die Herstellung von Supplementen wie Nahrungsergänzungsmittel, ergänzende bilanzierte Diät, Diätikum bekannt sind.

Üblicherweise besteht die Trägergrundlage bei festen oralen Verabreichungsformen (Tablette, Kapsel, Dragee) aus üblichen, auch pharmazeutisch zulässigen; Tablettierhilfsstoffen wie Sprengmittel, Füllstoffe, Fließmittel, ggf. auch Lösehilfen wie Zitronensäure, Hydrogencarbonat (z. B. für Brausetabletten), Flüssigkeiten wie Wasser, (Frucht)Säfte, Kohlenhydrat-Protein- Mischungen (für Riegel) oder z. B. aus Gelatineweichkapseln, in die die Zusammensetzung inkorporiert wird. Besonders konzipierte Darreichungsformen sind hier nicht erforderlich, es werden die jeweils nach dem Stand der Technik bekannten Methoden und Produkte angewendet.

### Herstellung

Die erfindungsgemäße Zusammensetzung zur beschriebenen wie vor allem enteralen Anwendung wird in an sich bekannter Weise hergestellt, indem man das System K, enthaltend KA, KP und KE herstellt durch Mischen der einzelnen gewünschten Substanzen, und dieses sodann mit dem System S, ausgewählt aus S1, S2, S3, S5 oder S6 in geeigneter Weise gegebenenfalls unter Verwendung jeweils geeigneter Zusatzstoffe für die gewünschte orale Verabreichungsform wie Träger, Füllstoffe, Tablettierhilfsmittel wie genannt, auf an sich bekannte Weise vermischt und homogenisiert. Methoden zur Herstellung derartiger Lebensmittel oder auch pharmazeutischer Mittel sind z. B. in Lehrbüchern beschrieben und allgemein bekannt.

Je nach Wirkstoff der Gruppe S können so pharmazeutische Zubereitungen oder auch Supplemente wie beschrieben erhalten werden.

Die Zusammensetzung kann insofern auch Teil eines Mittels in Form einer Zubereitung wie essbare Riegel etc. sein. Hierzu können Nahrungskomponenten, ausgewählt aus Aminosäuren, Kohlenhydraten, Fetten, die für die menschliche oder tierische Nahrung geeignet sind, z. B. Kohlenhydrat - Protein- Mischungen, gewählt werden. Bekannte Einzelkomponenten sind z. B. Fruchtsaft, Nektar, Fruchtmus wie Apfelsaft, Orangensaft, Apfelmus. Weiterhin bekannte Einzelkomponenten sind Getreideprodukte wie Weizen- Roggen-, Hafermehl, Maissirup, Milcheiweiß, Molke, Lecithin, Milchzucker. Je nach Wahl derartiger Vertreter der genannten Einzelkomponenten, können wie erwähnt Riegel, Flüssigkeiten, Drinks, z. B. Kraftdrinks (vorzugsweise nicht sirupösl wässrig sirupös), Brausetablette oder auch bilanzierte diätetische Ergänzungsmittel dieser Art hergestellt werden.

Dazu wird die wie beschrieben bereitete Zusammensetzung mit einer oder mehrere Nahrungskomponenten auf an sich bekannte Weise gemischt, und die gewünschte Form erhalten durch übliche Herstellungsverfahren.

### Anwendung

Die erfindungsgemäßen Zusammensetzungen werden insbesondere für die systemische bzw. enterale Anwendung konzipiert. Dabei sind orale Verabreichungsformen von Zusammensetzungen aus dem Aktiv- Wirksystem K + System S besonders geeignet.

Besonders geeignete Verabreichungsformen sind Gelatinekapseln, z. B. Weichgelatinekapsel, sowie Tabletten.

Aufgrund der besonderen Kombination der erfindungsgemäßen Zusammensetzung werden insgesamt stabile Produkte erhalten, deren Wirkungsweise unabhängig von der Darreichungsform erzielt werden kann.

Insbesondere kann diese Zusammensetzung bei strukturellen Veränderungen und funktionellen Störungen der betreffenden Organe, insbesondere des Haares, der Nägel, der Haut, Muskeln, des Gastrointestinaltrakts, der cerebralen Fähigkeiten, des immunologischen Systems als Supplement (nicht therapeutisch) wie Nahrungsergänzungsmittel, Diätikum, ergänzende bilanzierte Diät oder als pharmazeutisches Mittel bzw. zur Herstellung eines pharmazeutischen Mittels eingesetzt werden.

Diese Zusammensetzung kann wie erwähnt eingesetzt werden zur oralen Behandlung oder Prävention von strukturellen Veränderungen und funktionellen Störungen der betreffenden Zielorgane wie Haut, Nägel, Haare, Cerebrum, Muskeln und des immunologischen Systems, Gastrointestinaltrakt von Säugern, insbesondere Menschen.

Zur Herstellung von Kapseln oder Tabletten werden hierfür gebräuchliche Hilfsstoffe wie Lactose, Saccharose, Sorbit, Talkum, Stearinsäure, Magnesiumstearat, Gummi arabicum, Kartoffelstärke, Gelatine, PVP, Glycerin, Hydroxypropylmethylcellulose, Maltodextrin, Konservierungsmittel sowie übliche Materialien für Überzüge wie PEG 6000, Maisstärke, Zucker, Talkum, Farbstoffe in an sich bekannter Weise eingesetzt. Wenn per os verabreicht wird, kann die beschriebene Zusammensetzung z. B. vermischt werden mit Lactose, Saccharose, Stärkepulver, Celluloseester oder Alkansäuren, Cellulosealkylester, Talk, Stearinsäure, Magnesiumstearat, Magnesiumoxid, Natrium- und Calciumsalzen von Phosphor- und Schwefelsäuren, Gelatine, Akaziengummi, Natriumalginat, Glykole, Polyvinylpyrrolidon und/oder Polyvinylalkohol und dann tablettiert oder verkapselt werden. Die Zusatzstoffe und Tablettierungsverfahren sind allgemein bekannt und z.B. im aktuellen Europäischen Arzneibuch beschrieben. Insbesondere können sich die strukturellen Veränderungen und funktionelle Störungen auf Schwächezustände z. B. nach Krankheiten oder besonderen Belastungen (Stress), Alterserscheinungen oder auch Mangelerscheinungen beziehen.

Die Menge des verabreichten Mittels und der Dosierungsplan zur Prävention oder Behandlung eines wie oben beschriebenen Zustandes mit der erfindungsgemäßen Zusammensetzung hängen bei einer medizinischen Indikation von einer Vielzahl von Faktoren ab, einschließlich dem Alter, Gewicht, Geschlecht und Zustand des Patienten, der Schwere der Störung, der Verabreichungsroute und der jeweiligen verwendeten Verbindung, und kann deshalb weitestgehend schwanken. Die erforderliche oder gewünschte Dosis kann auf ein- oder mehrere Male täglich, insbesondere 1 bis 3 mal verteilt werden.

Bei der Verabreichung als Supplement werden hierfür allgemein übliche Mengen vorgeschlagen, welche auf ein- bis mehrmals, insbesondere 1 bis 3 mal oder 1 bis 2 mal täglich verteilt werden können. Diese können in fester Form, z. B. als Tablette, Kapsel, Dragee, oder als Riegel bzw. eine entsprechende Menge gelöst in Säften vorliegen.

### Beispiele

Die Erfindung wird anhand der nachfolgenden Beispiele 1 bis 4 näher erläutert. Die Wirksamkeit der erfindungsgemäßen Zusammensetzung wird anhand des nachfolgenden Beispiels 5 gezeigt

### Beispiel 1

### Dragee mit Wirksystem S1 (Haut)

Durch Vermischen der nachfolgenden Substanzen und Verpressen unter üblichen Bedingungen wurde ein erfindungsgemäßes Dragee erhalten aus:
I. Kapillar- Wirksystem K:
   1) Mittel aus der Gruppe KA: L-Arginin-Hydrochlorid:
   2) Mittel aus der Gruppe KP: Folsäure; Pyridoxin-HCL (B6);
      Cyanocobalamin (B12); Tocopherol (E); Traubenkemöl:
   3) Mittel aus der Gruppe KE: Q10:
II: Spezifisches Wirksystem S
   S1:Thiaminnitrat (B1), Calciumpantothenat (B5), Kieselerde,
III: Zusatzstoffe: Sojalecithin, Sojaöl, Gelatine, Glycerin

### Beispiel 2

### Tablette, mit Wirksystem S1 (Haare)

Durch Vermischen der einzelnen Inhaltsstoffe und Zusatzstoffe auf bekannte Weise wurde eine Tablette wie folgt erhalten:
I. Kapillar- Wirksystem K
   1) KA: L-Arginin-Hydrochlorid
   2) KP: Johannisbeersamenöl
   3) KE: Nicotinsäureamid (Niacin, B3)
II: Spezifisches Wirksystem S
   S1: Cystein, Methionin, Biotin, Zinkoxid
III. Zusatzstoffe: Lactose, Magnesiumstearat, Aerosil

### Beispiel 3 (nicht gemäß der Erfindung)

### Hartgelatinekapsel mit Wirksystem S3 (Skelett)

Auf bekannte Weise wurde eine Hartgelatinekapsel mit folgenden Substanzen erhalten:
I. Kapillar- Wirksystem K
   KA: L-Arginin-Hydrochlorid
II: Spezifisches Wirksystem S
   S3: Cholecalciferol (D3), Phyllochinon (K1), Calciumcarbonat, Kupfercarbonat
III. Zusatzstoffe: Gelatine, Magnesiumstearat, Talkum, Aerosil.

### Beispiel 4

### Weichgelatinekapsel, Wirksystem S1

Durch Vermischen der einzelnen Inhaltsstoffe auf bekannte Weise wurde eine Weichgelatinekapsel wie folgt erhalten:
I. Kapillar- Wirksystem K
   KA: L-Arginin-Hydrochlorid;
   KP: Vitamin E, Traubenkernöl, Leinöl;
   KE: Coenzym Q10; Magnesiumsalz; Nicotinsäureamid;
II. Vitamin A Palmitat; Biotin,; Zinksalz; Vitamin C Ascorbat.
III. Zusatzstoffe: Glycerin, Gelatine, Sorbit.

### Beispiel 5

### Anwendungsbeispiel

60 freiwillige ausgesuchte hautgesunde Probandinnen, je 15 pro Gruppe, erhielten über einen Zeitraum von 6 Monaten eine Vitalstoffkombination wie in der nachfolgenden Tabelle beschrieben, bzw. eine Kombination, enthalten nur die Kombination K bzw. S gemäß Tabelle bzw. Placebo (Gruppe K+S, K, S, Kontrolle) täglich morgens und abends eine Weichgelatinekapsel, mit den unten angegebenen Substanzen.

Die Überlegenheit der Kombination eines spezifischen Wirksystems "S" mit einem Kapillar-Wirksystem "K" läßt sich anhand dieses Anwendungsbeispiels eindrucksvoll zeigen. Bei diesem Verfahren wird bei freiwilligen Probanden ein Hautareal mit ultraviolettem Licht bestrahlt und anschließend die Photonenemission aus der bestrahlten Haut mittels "Single Photon Counting" gemessen. Je geringer die Photonenemission um so besser der Schutz der Haut vor oxidativen Schäden. Nach Einnahme der jeweiligen untersuchten Zusammensetzung K, bzw. S bzw. K+S wird ein Effekt erreicht, der sich UV-Licht- protektiv messbar nachweisen ließ. Die schützenden Eigenschaften sind bei der Kombination K+S überraschender Weise ausgeprägter als bei den Einzelkomponenten K, S und der Kontrollgruppe. Diese Tatsache dient als Wirksamkeitsnachweis für die überlegene Formulierung gemäß der erfindungsgemäßen, auch als Micro Nutri-Targeting- System bezeichenbaren Kombination.

### Methodenbeschreibung:

Zu Beginn und am Ende der Einnahme der jeweiligen Zusammensetzung wurde auf dem Rücken vor Studienbeginn ausgesuchter hautgesunder Probandinnen die sogenannte ICL-S (induced chemiluminescence of human skin) gemessen.

Hierbei wird eine Kuppel aus Flüssigkeits-Lichtwellenleitern mit einem zentral angeordneten Lichtwellenleiter zur Bestrahlung in einem optimierten Abstand und Winkel zur Haut lokalisiert, ohne diese zu berühren. Die Lichtwellenleiter befinden sich in einem Messkopf, der fest auf die Haut gedrückt wird. Diese Anordnung erlaubt einen definierten Photonentransfer von der Haut zum Detektor und kompensiert Einflüsse wie Schwitzen oder Bewegungen der Testperson.

Je Person wurden vier Untersuchungsareale zwei Minuten mit UVA (320 ≤ λ ≤ 400 nm) bestrahlt (Bestrahlungsenergie 10mW/cm²_{,} λmax = 350 nm). Unmittelbar nach diesem UV-Stress wurde die Photonenemission der Haut mittels Single-Photon-Counting über einen Zeitraum von 200 s gemessen. Die Menge an freigesetzten Photonen korreliert dabei mit dem durch UVA-Strahlung ausgelösten "oxidativen Stress" [Sauermann G, Mei WP, Hoppe U, Stäb F: Ultraweak photon emission in vivo: Influence of topically applied antioxidants on human skin. Mehtods Enzymol 199; 300:419-4281.

Der Mittelwert der integralen Photonenemission sowie die mittleren Abklingkinetiken der von der Haut emittierten Photonen wurden vor und nach Einnahme der Kombination berechnet und auf signifikante Unterschiede getestet.

Unterschiede im Hautkolorit wurden rechnerisch berücksichtigt.

Im Rahmen der vorliegenden Untersuchung konnte belegt werden, dass die Intervention mit der erfindungsgmäßen Kombinationen K +S zu einer Stärkung der antioxidativen Schutzfunktionen der Haut führen. Hierbei wurden nicht, wie sonst üblich, oxidierte Metabolite indirekt nachgewiesen, es erfolgte vielmehr eine direkte Messung der UV-induzierten Chemilumineszenz der Haut bei den einzelnen Probandinnen. Auf diese Weise wurden mittels einer physikalischen Messmethodik funktionell relevante Ergebnisse erhalten, die einen unmittelbaren Rückschluss auf den Schutz der Haut gegen die von exogenen Noxen ausgehenden Schäden erlauben. Diese Verbesserung der Schutzfunktion der Haut ist nicht nur wesentlich im Sinne eines dermalen Anti-Aging-Effektes, sondern auch im Sinne der Prävention vieler altersabhängiger Erkrankungen der Haut, die mit oxidativen Belastungen zusammenhängen.

Da infolge der UVA-Belastung durch Sonnenstrahlen Schäden der mitochondrialen DNA auftreten, die nicht nur über Jahre bestehen bleiben können, sondern deren Gehalt in der Haut durch einen einmal induzierten Mechanismus auch ohne weitere Bestrahlung weiter ansteigen kann, werden Antioxidantien zur Vermeidung von Photo-Aging im Sinne einer Dauerbehandlung und nicht nur für die Zeit der Sonnenexposition empfohlen [GD-Symposium: Wirkungen von Dermakosmetika. Dermotopics 1, 2002; 93:9-12].

In keinem Fall sollte allerdings die Verwendung von Antioxidantien zu einem übermäßigen Sonnengenuß verleiten.

Die jeweils eingesetzte Kombination K, bzw. S bzw. K+S enthielt folgende Substanzen (Mikronährstoffe) nach Art und Menge wie aufgeführt:

| Modul | Substanz (Mikronährstoff) | Tagesdosis |
|---|---|---|
| K | Kapillar-Aktivatorsystem KA Gefäß-Erweiterung, Blutfluss-Steigerung | |
| Kapillar-Wirk-System | L - Arginin | 250 mg |
| | Kapillar-Protektorsystem KP Endothelstablisierung, Gefäßschutz, Lipoproteinschutz | |
| | Vitamin E | 42 mg |
| | Traubenkemöl | 514 mg |
| | enthält mind. Ω-6-Fettsäuren | 360 mg |
| | Leinöl | 178,8 mg |
| | enthält mind. Ω-3-Fettsäuren | 90,0 mg |
| | Kapillar-Energieversorgungssystem KE Redoxsysteme und Cofaktoren bei der Energiebereitstellung, Energieträger | |
| | Coenzym Q₁₀ | 5 mg |
| | Magnesium | 300 mg |
| | Niacin | 39 mg |
| S Spezifisches Wirk-System | Spezifisches Wirksystem S Spezifisches Wirksystem Haut S | |
| | Vitamin A | 0,8 mg |
| | Biotin | 0,18 mg |
| | Zink | 9,0 mg |
| | Vitamin C | 100 mg |

Die erhaltenen ICL- Werte sind in Figur 1 in Abhängigkeit von der Zeit dargestellt.

Wie hieraus ersichtlich ist, führt die erfindungsgemäße Vitalstoff-Kombination aus K +S zu einer überraschenden Stärkung der antioxidativen Schutzfunktionen der Haut. Dabei zeigte sich unerwarteter Weise ein überadditiver Effekt. Dies ist umso vorteilhafter, da die infolge der UVA-Belastung durch Sonnenstrahlen aufgetretenen Schäden der mitochondrialen DNA über Jahre bestehen bleiben können und darüber hinaus auch deren Gehalt in der Haut durch einen einmal induzierten Mechanismus auch ohne weitere Bestrahlung weiter ansteigen kann. Antioxidative Mittel sind daher besonders wünschenswert zur Vermeidung von Photo-Aging, insbesondere auch im Sinne einer Dauerbehandlung und nicht nur für die Zeit der Sonnenexposition.

## Patentansprüche

1. Zusammensetzung zur oralen Anwendung mit einer anwendungsbezogenen Wirkstoffdifferenzierbarkeit, **dadurch gekennzeichnet, dass** sie besteht aus:
I.) einem Kapillar- Wirksystem K aus:
- einem Kapillar - Aktivatorsystem KA, ausgewählt aus einem oder mehreren Mitteln aus Gingko - Extrakt, Mäusedorn Extrakt, L- Arginin-Hydrochlorid;
- einem Kapillar-Protektorsystem KP ausgewählt aus einem oder mehreren Mitteln aus Folsäure, Pyridoxinhydrochlorid, Cyanocobalamin, Tocopherylacetat, omega-3- und 6-Fettsäure-haltigen Ölen, und
- einem Kapillar - Energieversorgungssystem KE, ausgewählt aus einem oder mehreren Mittel, ausgewählt aus Nicotinsäure und dessen Derivaten, insbesondere Nicotinsäureamid, Magnesiumoxid, Co- Enzym Q10, Coffein, Glukose;
II.) einem spezifischen Wirksystem S, ausgewählt aus:
- einem oder mehreren Haut-und -Anhangsgebilde beeinflussenden Mitteln S1, ausgewählt aus Retinol, Retinolacetat/-palmitat (Vitamin A), Thiaminnitrat (B₁), Biotin, Ca-pantothenat (B₅) (B-Vitamine), Kieselerde, Kieselsäure, Zinkoxid / -sulfat /-gluconat, pflanzlichen Extrakten aus Algen, Papaya, Schachtelhalm, Kamille, Aloe vera, den Aminosäuren Cystein, Methionin, Gammalinolensäure (GLA), Linolsäure, Vitamin C (Ascorbinsäure, Calciumascorbat/Natriumascorbat, Kaliumascorbat), Finasteroid, Minoxidil, Isotretinoin, Prednisolon; oder
- einem oder mehreren cerebral wirksamen Mitteln 52, ausgewählt aus Ginseng, Paranuß, Wassernabel, Frauenwurz, den Aminosäuren, Glutaminsäure, Tyrosin, Tryptophan, Uridin; Docosahexaensäure (DHA), Phosphatidylserin , Phosphatidylinositol, Cholin, Lecithin; Amantadin, Dopamin; oder
- einem oder mehreren immunologisch wirksamen Mitteln S3, ausgewählt aus Carotinoiden wie Lycopin, Natriumselenat, pflanzlichen Extrakte aus Grüntee, Echinacea, Schisandra, Astrag alus, Reishi, Shiitake, Maistake, Wiesenklee, Soja, Katzenkralle, Traubenkern, Brokkoli, Tomate, Zwiebel, Blumenkohl, Citrus-Biofla vonoide, Buchweizen, Grapefruit, den Aminosäuren Threonin, Histidin, Glutathion, Superoxiddismutase; oder
- einer oder mehreren oder Muskel-wirksamen Substanzen S5, ausgewählt aus Inositol (B₈), Aminosäuren wie Leucin, Isoleucin, Valin, Dantrolen, Tetrazepam;
- oder einer oder mehreren den Gastrointestinaltrakt beeinflussenden Substanzen S6, ausgewählt aus α-Linolensäure, Kaliumsalzen wie Kalium-carbonat/ hydro-gencarbonat/-citrat/-chlorid/-gluconat/-lactat/-phosphat, Chromsalzen wie Chromchlorid/-picolinat/-sulfat, Famotidin, Metoclopramid; sowie
III.) 0,1 bis 90% galenisch oder pharmazeutisch übliche Zusatzstoffen.

2. Zusammensetzung nach Anspruch 1, **dadurch gekennzeichnet, dass** L-Argininhydrochlorid als System KA vorliegt.

3. Zusammensetzung nach Anspruch 1 oder 2, **dadurch gekennzeichnet, dass** sie in Form einer Tablette, eines Dragees, oder einer Kapsel vorliegt.

4. Zusammensetzung nach einem der Ansprüche 1 bis 3, **dadurch gekennzeichnet, dass** ein spezifisches System S1, S2 oder S3 vorliegt.

5. Zusammensetzung nach einem der Ansprüche 1 bis 4, **dadurch gekennzeichnet, dass** sie eine Weichgelatinekapsel aus einem Kapillar- Wirksystem K aus L-Arginin-Hydrochlorid; Vitamin E, Traubenkernöl, Leinöl; Q10; Magnesiumsalz; Nicotinsäureamid; einem spezifischen Wirksystem S1 aus Vitamin A Palmitat; Biotin; Zinksalz; Vitamin C -Ascorbat; und Zusatzstoffen aus Glycerin, Gelatine, Sorbit darstellt.

6. Verwendung einer Zusammensetzung gemäß einem der Ansprüche 1 bis 4 als Nahrungsergänzungsmittel, Diätikum, oder ergänzende bilanzierte Diät.

7. Verwendung gemäß Anspruch 6 in Form eines Dragees, einer Kapsel, einer Tablette oder eines Riegels.

8. Verwendung einer Zusammensetzung nach einem der Ansprüche 1 bis 4 zur Herstellung eines pharmazeutischen Mittels.

## Claims

1. Composition for oral administration with an application-relevant drug differentiability, **characterised in that** it consists of
I.) a capillary active system K of:
- a capillary activator system KA, selected from one or more agents from gingko extract, Butcher's broom extract, L-arginine hydrochloride,
- a capillary-protective system KP selected from one or more agents from folic acid, pyridoxine hydrochloride, cyanocobalamine, tocopheryl acetate, oils containing omega-3 and omega-6 fatty acids, and
- a capillary energy supply system KE, selected from one or more agents, selected from nicotinic acid and its derivatives, especially nicotinamide, magnesium oxide, Coenzyme Q10, caffeine, glucose;
II.) a specific active system S, selected from:
one or more agents S1 that affect skin and its adnexa, selected from retinol, retinol acetate/palmitate (vitamin A), thiamine nitrate (B₁), biotin, calcium pantothenate (B₅) (B-vitamins), silica, silicic acid, zinc oxide/- sulfate/-gluconate, vegetal extracts of algae, papaya, horsetail, camomile, aloe vera, the amino acids cysteine, methionine, gamma linolenic acid (GLA), linoleic acid, vitamin C (ascorbic acid, calcium ascorbate/sodium ascorbate, potassium ascorbate), finasteroid, minoxidil, isotretinoin, prednisolone; or
one or more cerebrally active agents S2, selected from ginseng, brazil nut, ρennywort, cohosh, the amino acids, glutamic acid, tyrosine, tryptophan, uridine, docosahexaenic acid (DHA), phosphatidylserine, phosphatidylinositol, choline, lecithin, amantadine, dopamine; or
one or more immunologically active agents S3, selected from carotenoids such as lycopene, sodium selenate, vegetal extracts of green tea, Echinacea, Schisandra, Astragalus, Reishi, shiitake, maitake, red clover, soy, cat's claw, grape seed, broccoli, tomato, onion, cauliflower, citrus bioflavonoids, buckwheat, grapefruit, the amino acids threonine, histidine, glutathione, super oxide dismutase; or
ne or more or substances S5 that affect muscles, selected from inositol (B₈), amino acids such as leucine, isoleucine, valine, dantrolene, tetrazepam;
or one or more of the substances S6 that affect the gastro-intestinal tract,
selected from α-linolenic acid, potassium salts such as potassium carbonate/hydrogen carbonate/citrate/rhioride/gluconatellactate/ phosphate, chromium salts such as chromium chloride/ picolinate/sulfate, famotidine, metoclopramide; and
III.) 0.1 to 90% of customary galenical or pharmaceutical additives.

2. Composition according to claim 1, **characterised in that** L-argininehydrochloride is present as the system KA.

3. Composition according to claim 1 or 2, **characterised in that** it is in the form of a tablet, a coated tablet or a capsule.

4. Composition according to one of claims 1 to 3, **characterised in that** a specific system S1, 52 or 53 is present.

5. Composition according to one of claims 1 to 4, **characterised in that** it represents a soft gelatine capsule of a capillary active system K of L-arginine hydrochloride; vitamin E, grape seed oil, linseed oil; Q10; magnesium salt; nicotinamide; a specific active system S1 of vitamin A palmitate; biotin; zinc salt; vitamin C ascorbate; and additives from glycerine, gelatines, sorbitol.

6. Use of a composition according to one of claims 1 to 4 as a dietary supplement, dietary component, or supplemental balanced diet.

7. Use according to claim 6 in the form of a coated tablet, a capsule, a tablet or a bar.

8. Use of a composition according to one of claims 1 to 4 for the manufacture of a pharmaceutical.

## Revendications

1. Composition pour l'application orale comprenant une différentiabilité de substance active rapportée à l'application, **caractérisée en ce qu'**elle est constituée par :
I.) un système actif K de type capillaire constitué par :
- un système d'activation capillaire KA choisi parmi un ou plusieurs agents choisis parmi un extrait de ginkgo, un extrait d'épine de rat, le chlorhydrate de L-arginine ;
- un système de protection capillaire KP choisi parmi un ou plusieurs agents choisis parmi l'acide folique, le chlorhydrate de pyridoxine, la cyanocobalamine, l'acétate de tocophéryle, des huiles contenant des acides gras oméga 3 et 6 ; et
- un système d'alimentation d'énergie capillaire KE choisi parmi un ou plusieurs agents choisis parmi l'acide nicotinique et ses dérivés, en particulier l'amide de l'acide nicotinique, l'oxyde de magnésium, la coenzyme Q10, la caféine, le glucose ;
II.) un système actif spécifique S choisi parmi :
un ou plusieurs agents S1 influençant la peau et ses appendices, choisis parmi le rétinol, l'acétate / le palmitate de rétinol (vitamine A), le nitrate de thiamine (B₁), la biotine, le pantothénate de calcium (B₅) (vitamines B), la silice, l'acide silicique, l'oxyde / le sulfate / le gluconate de zinc, des extraits végétaux d'algues, de papaye, de prêle, de camomille, d'aloe vera, les acides aminés : cystéine, méthionine ; l'acide γ-linolénique (GLA), l'acide linoléique, la vitamine C (acide ascorbique, ascorbate de calcium 1 ascorbate de sodium, ascorbate de potassium), le finastéride, le minoxidil, l'isoirétinoïne, la prednisolone ; ou
un ou plusieurs agents S2 à activité cérébrale, choisis parmi le ginseng, la noix de Brésil, l'écuelle d'eau, l'herbe de saint Christophe, les acides aminés: acide glutamique, tyrosine, tryptophane, uridine; l'acide docosahexaénoïque (DHA), la phosphatidylsérine, le phosphatidylinositol, la choline, la lécithine ; l'amantadine, la dopamine; ou
un ou plusieurs agents S3 à activité immunologique, choisis parmi des carotinoïdes tels que le lycopène, le sélénate de sodium, des extraits végétaux de thé vert, d'échinacée, de schisandra, d'astragale, de reishi, de shiitake, de maitake, de trèfle des prés, de soja, de griffe de chat, de pépin de raisin, de brocoli, de tomate, d'oignon, de chou-fleur, de bioflavonoïdes de citrus, de sarrasin, de pamplemousse ; les acides aminés : thréonine, histidine ; le glutathion ; la superoxyde dismutase ; ou
une ou plusieurs substances 55 à activité musculaire, choisies parmi l'inositol (B₈), des acides aminés tels que la leucine, l'isoleucine, la valine ; le dantrolène, le tétrazépam ; ou
une ou plusieurs substances S6 influençant le tractus gastro-intestinal, choisies parmi l'acide α-linolénique, des sels de potassium tels que le carbonate / l'hydrogéno-carbonate / le citrate / le chlorure / le gluconate / le lactate / le phosphate de potassium, des sels de chrome tels que le chlorure / le picolinate / le sulfate de chrome, la famotidine, le métoclopramide ; ainsi que
III.) de 0,1 à 90 % d'additifs habituels du point de vue galénique ou pharmaceutique.

2. Composition selon la revendication 1, **caractérisée en ce que** le chlorhydrate de L-arginine est présent à titre de système KA.

3. Composition selon la revendication 1 ou 2, **caractérisée en ce qu'**elle se présente sous la forme d'un comprimé, d'une dragée ou d'une capsule.

4. Composition selon l'une quelconque des revendications 1 à 3, **caractérisée en ce qu'**un système spécifique S1, S2 ou S3 est présent.

5. Composition selon l'une quelconque des revendications 1 à 4, **caractérisée en ce qu'**elle représente une capsule de gélatine molle d'un système actif K de type capillaire de chlorhydrate de L-arginine ; de la vitamine E, de l'huile de pépin de raisin, de l'huile de lin ; du Q10 ; du sel de magnésium ; de l'amide de l'acide nicotinique ; d'un système actif spécifique S1 de palmitate de vitamine A ; de la biotine ; du sel de zinc ; de l'ascorbate de vitamine C ; et par des additifs choisis parmi le glycérol, la gélatine, le sorbitol.

6. Utilisation d'une composition selon l'une quelconque des revendications 1 à 4, à titre de complément alimentaire, d'agent diététique ou de régime équilibré complémentaire.

7. Utilisation selon la revendication 6, sous la forme d'une dragée, d'une capsule, d'un comprimé ou d'une barre.

8. Utilisation d'une composition selon l'une quelconque des revendications 1 à 4, pour la préparation d'un agent pharmaceutique.
